# EUROPEAN PATENT APPLICATION

(11) **EP 3 597 173 A1**
(43) Date of publication of application: **22.01.2020**
(21) Application number: 18767710.9
(22) Date of filing: 02.03.2018
(51) Int. Cl.: A61K 8/73, A61K 8/02, A61K 8/34, A61Q 1/00, A61Q 19/00, C08B 16/00

(54) **METHOD FOR PASTING CELLULOSE FILM ONTO SKIN, AND COSMETIC FOR PASTING USE**

(30) Priority: 13.03.2017 JP 2017046867
(71) Applicant: Panasonic Intellectual Property Management Co., Ltd., Osaka-shi, Osaka 540-6207 (JP)
(72) Inventor: KUSUKAME Haruka, Osaka-shi, Osaka 540-6207 (JP); KAWASHIMA Tomoko, Osaka-shi Osaka 540-6207 (JP); AOKI Takahiro, Osaka-shi Osaka 540-6207 (JP); TANIIKE Yuko, Osaka-shi Osaka 540-6207 (JP)
(74) Representative: Grünecker Patent- und Rechtsanwälte PartG mbB
(86) International application number: PCT/JP2018/007976
(87) International publication number: WO 2018/168518

(57) **Abstract**

The present disclosure provides a method for stably sticking a sheet to skin for a long time without giving a skin stress and strange feeling of appearance. In the present disclosure, a self-supporting cellulose membrane having a thickness of not less than 20 nm and not more than 1,300 nm and containing regenerated cellulose having a weight average molecular weight of not less than 150,000 is used, and after applying to skin an aqueous cosmetic agent containing water and at least one polyhydric alcohol and having a content of glycerin of the polyhydric alcohol of 0% by weight to 15% by weight, the cellulose membrane is stuck to the position of the skin on which the cosmetic agent is applied.

## Description

### TECHNICAL FIELD

The present disclosure relates to a method for sticking a self-supporting cellulose membrane to skin, and a cosmetic agent for sticking to skin.

### BACKGROUND ART

In cosmetic and medical fields, a sheet that is to be stuck to skin to conceal a scar on the skin or make blotches and wrinkles inconspicuous, and is capable of achieving makeup or skin care has been investigated. When the sheet has a large thickness, the sheet stuck to skin gives strange feeling of appearance. The poor stickiness of such a sheet to skin requires an adhesive for sticking, to result in large stress on the skin. On the other hand, it is known that the sheet having a thickness of several hundred nanometers or less exhibits high adhesiveness, and can be stuck to skin without use of an adhesive. For example, PTL 1 enables sticking to skin without use of an adhesive by using a self-supporting thin membrane including polylactic acid as a sheet material.

### Citation List

### Patent Literature

PTL 1: WO 2008/050913

### SUMMARY OF THE INVENTION

### TECHNICAL PROBLEM

However, a hydrophobic material such as polylactic acid has such a problem that the low water vapour transmissivity of the sheet material may cause a stress such as stuffiness to skin when the sheet material is stuck to the skin for a long time. An object to be attained by the present invention is to provide a method for sticking a cellulose thin membrane having high water vapour transmissivity to skin stably for a long time without using a pressure sensitive adhesive.

### SOLUTION TO PROBLEM

The present invention provides a method for sticking a sheet, the method comprising applying a cosmetic agent containing water and at least one kind of polyhydric alcohol to skin, and then placing a self-supporting cellulose membrane having a thickness of not less than 20 nm and not more than 1,300 nm and containing regenerated cellulose having a weight average molecular weight of not less than 150,000 on the skin, wherein an amount of glycerin of the polyhydric alcohol contained in the cosmetic agent is not less than 0% by weight and not more than 15% by weight.

### ADVANTAGEOUS EFFECT OF INVENTION

According to the method for sticking a sheet according to the present disclosure, it is possible to stick a cellulose thin membrane having high water vapour transmissivity to skin stably for a long time without using a pressure sensitive adhesive.

### BRIEF DESCRIPTION OF DRAWINGS

FIG. 1 is a schematic view showing a state that cellulose membrane 100 is stuck to skin 200 on which cosmetic agent 300 has been applied.
FIG. 2 is a schematic perspective view showing an example of a cellulose membrane retaining a component that acts on living organism or protects living organism.
FIG. 3 is a schematic perspective view showing multilayer sheet 100A having cellulose membrane 100.
FIG. 4 is a schematic perspective view showing a state that a part of protective layer 101 is peeled off from one principal face of cellulose membrane 100.
FIG. 5 is a view showing a use example in which cellulose membrane 100 is stuck to a part of a face.
FIG. 6 is a view showing a state that multilayer sheet 100A is stuck to skin 200.

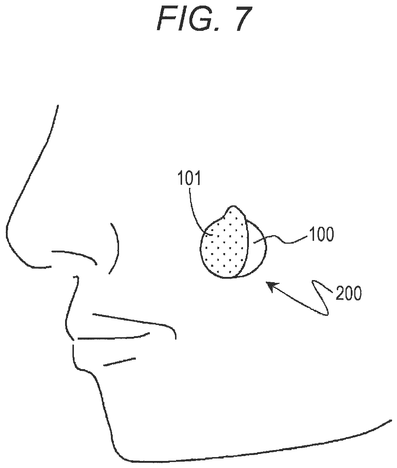

FIG. 7 is a view showing a state in a course of peeling off protective layer 101 from cellulose membrane 100 on skin 200.
FIG. 8 is a schematic perspective view showing multilayer sheet 100B having cellulose membrane 100, protective layer 101 and second protective layer 102.
FIG. 9 is a schematic perspective view showing a state that a part of protective layer 101 is peeled off from cellulose membrane 100 of multilayer sheet 100B.
FIG. 10 is a view showing a state that a laminate of cellulose membrane 100 and second protective layer 102 is stuck to skin 200.
FIG. 11 is a view schematically showing a state that colored cellulose membrane 100b is stuck to skin 200.

### DESCRIPTION OF EMBODIMENT

### UNDERLYING KNOWLEDGE FORMING BASIS OF THE PRESENT DISCLOSURE

The present inventors have investigated for a sheet that gives no skin stress such as stuffiness or rash when the sheet is stuck to the skin. As a result of investigation, the present inventors have found that by sticking to skin a self-supporting cellulose membrane having high water vapour transmissivity and a thickness of several micrometers or less, the self-supporting cellulose membrane being made up of amphipathic cellulose as a sheet material, it is possible to stick the membrane to the skin without using an adhesive, and it is possible to avoid a skin stress such as stuffiness or rash caused by the sheet. In cosmetic or medical use applications, it is necessary to stably stick a thin membrane to skin for a long time so as to sustain the effect of the thin membrane. It has been also found that when the cellulose membrane is stuck to skin, the cellulose membrane can be stuck while an aqueous liquid such as water or a lotion, or an oily cream is interposed between the cellulose membrane and the skin, and thus the cellulose membrane can be stuck to the skin for not less than 8 hours. However, there may be a case where the adhesiveness against frictional stimulation may be insufficient, or sticking for a longer time may be required. Then, the present inventors have investigated for a method for sticking a cellulose membrane that is less likely to result in peeling by frictional stimulation, and enables sticking for a longer time, and have found that by applying on skin a cosmetic agent in which a polyhydric alcohol component is mixed, and sticking the cellulose membrane after the cosmetic agent is applied between the cellulose membrane and the skin, it is possible to stick the cellulose membrane stably for a long time.

### EMBODIMENT

A method for sticking a sheet to skin according to the present embodiment is described below.

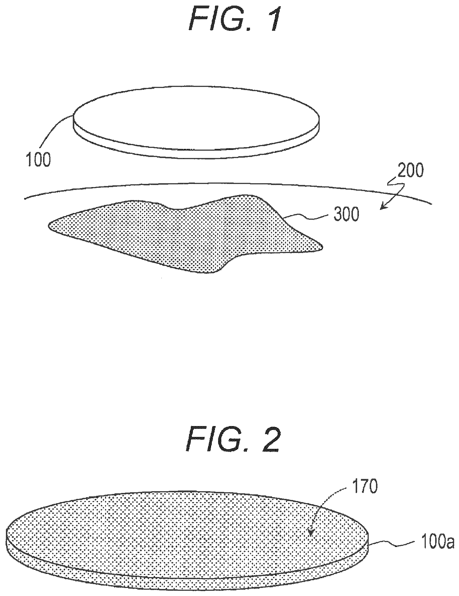

FIG. 1 is a schematic view showing the state that cellulose membrane 100 is stuck to skin 200 on which cosmetic agent 300 has been applied. As shown in FIG. 1, the method is a method of applying a cosmetic agent to skin, and then sticking a self-supporting cellulose membrane to a part where the cosmetic agent is applied.

### CELLULOSE MEMBRANE

A cellulose membrane according to an embodiment of the present disclosure is a cellulose membrane composed of regenerated cellulose having a weight average molecular weight of not less than 150,000. The cellulose membrane according to an embodiment of the present disclosure is a self-supporting thin membrane having a thickness of not less than 20 nm and not more than 1,300 nm.

In the present description, "self-supporting membrane" means a membrane capable of keeping the form of a membrane without a support, and means such a membrane that when a part of the membrane is held with, for example, fingers or tweezers, and the membrane is lifted, the entire membrane can be lifted without a support while the membrane is not damaged. In the present description, "regenerated cellulose" means cellulose lacking a crystal structure I that is peculiar to natural cellulose. The crystal structure of cellulose can be confirmed by an XRD pattern.

It is preferred that regenerated cellulose in the cellulose membrane according to an embodiment of the present disclosure is not chemically modified or derivatized for not less than 90% of the regenerated cellulose, more preferably for not less than 98% of the regenerated cellulose. Regenerated cellulose in the cellulose membrane may not be cross-linked.

As will be described later, the cellulose membrane according to an embodiment of the present disclosure can be used while it is stuck to skin of, for example, a face, an arm or the like. The cellulose membrane according to an embodiment of the present disclosure typically has an area of not less than 7 mm². The area of the cellulose membrane of not less than 7 mm² is advantageous because a large region can be covered when the membrane is stuck to the skin.

The cellulose membrane according to an embodiment of the present disclosure has, for example, a bulk density of not less than 0.3 g/cm³ and not more than 1.5g/cm³. A bulk density of not less than 0.3 g/cm³ is advantageous because the strength required for keeping the shape of the cellulose membrane can be ensured. It is also possible to make the cellulose membrane itself retain a component that acts on living organism or protects living organism, such as a cosmetic component or an active ingredient. For example, such a component can be retained in voids inside the membrane. In particular, when the cellulose membrane has a bulk density of lower than 1.5 g/cm³, which is the true density of cellulose, the cosmetic component or the like can permeate in the membrane more easily. The component that acts on living organism or protects living organism, such as a cosmetic component, may be retained in a solid state in voids inside the membrane, or may be dissolved and/or dispersed in a liquid, and retained in a state of dispersion or cream in voids inside the membrane.

The cellulose membrane according to an embodiment of the present disclosure can have a degree of crystallinity of not less than 0% and not more than 12%. When the degree of crystallinity is not more than 12%, the rate of the hydroxyl groups involved in formation of the crystal form is reduced, so that the adhesiveness to skin of the cellulose membrane can be improved.

The cellulose membrane according to an embodiment of the present disclosure can have a water vapour transmission rate (WVTR) of not less than 1 × 10⁴ g/m²·24 h. A cellulose membrane having a water vapour transmission rate of not less than 1 × 10⁴ g/m²·24 h is advantageous because uncomfortableness caused by stuffiness or the like can be reduced when cellulose membrane is stuck to skin.

The cellulose membrane according to an embodiment of the present disclosure can have a contact angle with water within the range of 0° to 30°. When a cellulose membrane has a contact angle within this range, the affinity between the surface of the membrane and water increases, and the cellulose membrane absorbs water on the skin quickly, so that stability and comfortableness when the sheet is stuck can be made more excellent.

The cellulose membrane according to an embodiment of the present disclosure may have a thickness of not less than 50 nm and not more than 1,000 nm. When the thickness is not less than 50 nm, higher strength is obtained, and handling of the cellulose membrane is further facilitated. The thickness of the cellulose membrane of not more than 1,000 nm is advantageous because the cellulose membrane stuck to skin is inconspicuous. The cellulose membrane may have a thickness of not less than 500 nm and not more than 1,000 nm. When the thickness is not less than 500 nm, a tear-proof cellulose membrane having higher strength is obtained. Also, it is possible to make the cellulose membrane retain a larger amount of an effective component (for example, cosmetic component). The cellulose membrane may have a thickness of not less than 100 nm and not more than 500 nm. A thickness of not less than 100 nm is advantageous for keeping the shape of the thin membrane. By making the thickness be not more than 500 nm, the adhesiveness of the cellulose membrane is further improved. Therefore, it is possible to stick the cellulose membrane to skin or other surface stably for a longer time. Also, further reduction in thickness of the cellulose membrane makes the cellulose membrane more inconspicuous on the skin.

The cellulose membrane according to an embodiment of the present disclosure has a tensile strength of not less than 23 MPa. The cellulose membrane having a tensile strength of not less than 23 MPa will not easily break even when it is stuck to skin, and the cellulose membrane can be stuck on the skin for a long time.

Hereinafter, an example of a method for producing a cellulose membrane according to an embodiment of the present disclosure will be described. First, cellulose is dissolved in a solvent to prepare a cellulose solution. In an embodiment of the present disclosure, cellulose having a weight average molecular weight of at least not less than 150,000 is used as cellulose to be dissolved in a solvent from the viewpoint of finally obtaining a cellulose membrane having a weight average molecular weight of not less than 150,000. The cellulose that can be used may be cellulose derived from plants such as pulp or cotton, or cellulose generated by organisms such as bacteria as long as the cellulose has a predetermined weight average molecular weight. By using cellulose having a weight average molecular weight of not less than 150,000, it is possible to provide a regenerated cellulose membrane having a thickness of not more than 1,300 nm (1.3 µm) and a self-supportable strength. It is advantageous that cellulose as a raw material has a concentration of impurities of not more than 5 wt%.

If the weight average molecular weight is too large, the viscosity of the solution is high and the solution is hard to be subject d to processing. Accordingly, the regenerated cellulose in the cellulose membrane that is finally obtained has a weight average molecular weight of preferably not more than 1,000,000, more preferably not more than 500,000, further preferably not more than 300,000. A weight average molecular weight of not more than 1,000,000 can be subjected to processing, and a weight average molecular weight of not more than 5,000,000,000 can be easily subjected to processing, and a weight average molecular weight of not more than 3,000,000,000 provides a more stable sheet with less variation.

As the solvent, a solvent containing at least an ionic fluid is used. By using a solvent containing at least an ionic fluid, it is possible to dissolve cellulose having a weight average molecular weight of not less than 150,000 in a relatively short time. An ionic fluid is a salt composed of an anion and a cation, and may show a liquid state at a temperature of not more than 150°C. As the ionic fluid that dissolves cellulose, an ionic fluid containing amino acid or alkyl phosphate ester can be used. By using such an ionic fluid as a solvent, it is possible to dissolve cellulose while suppressing decrease in molecular weight. In particular, since amino acid is a component existing in living organism, it can be said that an ionic fluid containing amino acid enables preparation of a regenerated cellulose membrane that is safer to living organism.

### COSMETIC AGENT

The cosmetic agent of the present disclosure is an aqueous cosmetic agent containing at least water and at least one kind of polyhydric alcohol, in which a content of glycerin of the polyhydric alcohol ranges from 0% by weight to 15% by weight. As a result, the drying speed of the cellulose membrane containing the cosmetic agent after being stuck to skin is suppressed, and contraction of the cellulose membrane is reduced, resulting that the adhesion to the skin can be improved. Also, the followability of the cellulose membrane to skin increases without remaining a large amount of oily component between the skin and the cellulose membrane after the cellulose membrane is stuck to the skin, and the cellulose membrane becomes less likely to be peeled off by external stimulation such as friction, and the cellulose membrane can be stuck to the skin stably for a long time.

The polyhydric alcohol used in the present invention can be selected from, but is not particularly limited to, diol alcohols, and examples of the diol alcohols include propanediol, propylene glycol, dipropylene glycol, 1,3-butylene glycol, 1,2-pentanediol, and 1,2-hexanediol, with propanediol, propylene glycol, and dipropylene glycol being particularly preferred. In both cases of containing only one kind of propanediol, propylene glycol, or dipropylene glycol, and containing glycerin in combination, the strange feeling of appearance of the cellulose membrane stuck to skin is reduced, and the adhesiveness is improved.

The mixing amounts of propanediol and glycerin used in the present invention preferably range from 5% by weight to 15% by weight for propanediol, and range from 5% by weight to 10% by weight for glycerin.

As the polyhydric alcohol used in the present invention, a combination of two kinds of diol alcohols may be used. It is particularly preferred that propanediol and 1,3-butylene glycol are contained.

In the present invention, it is preferred that, for example, ethanol is contained as a monohydric alcohol. This improves the antiseptic property of the cosmetic agent.

In the present invention, it is preferred that a water-soluble polymer is contained to improve the feeling of the cosmetic agent. Examples of the water-soluble polymer that can be used include natural water-soluble polymers, semi-synthetic water-soluble polymers, and synthetic water-soluble polymers.

Specific examples of the natural water-soluble polymers that can be used include plant-derived polymers such as gum arabic, gum tragacanth, galactan, guar gum, carob gum, karaya gum, carrageenan, pectin, agar, quince seed (marmelo), algae colloid (brown alga extract) or starch (rice, corn, potato, wheat); microorganism-derived polymers such as xanthan gum, dextran, succinoglucan, or pullulan; and animal-derived polymers such as collagen, casein, albumin, or gelatin.

Also, biologically derived polymer compounds such as hyaluronic acid, mucin, chondroitin sulfate, or soluble collagen may be used.

Examples of the semi-synthetic water-soluble polymers that can be used include starch polymers such as carboxymethyl starch, or hydroxypropyl starch; cellulose polymers such as methylcellulose, hydroxyethylcellulose, sodium cellulose sulfate, hydroxypropylcellulose, or carboxymethylcellulose (CMC); and alginate polymers such as sodium alginate, or propylene glycol alginate.

Examples of the synthetic water-soluble polymers that can be used include vinyl polymers such as polyvinyl alcohol, polyvinylmethyl ether, polyvinylpyrrolidone, or carboxyvinyl polymer (carbomer); polyoxyethylene polymers such as polyethyleneglycol; copolymeric polymers such as polyoxyethylene polyoxypropylene copolymer; acryl polymers such as sodium polyacrylate, or acrylate-alkyl methacrylate copolymer; and polyethylene imine.

In the present invention, a moisturizing agent other than the components as described above may be contained for keeping the moisture of the skin. Specific examples of the moisturizing agent include polyethylene glycol, sorbitol, xylitol, maltitose, sodium dl-pyrrolidone carboxylate, sodium lactate, and trimethylglycine.

Components other than those described above may be contained unless the effect of the present disclosure is not impaired. Examples of the other components include a surfactant, a pH regulator, a UV absorber, a UV scatterer, an aseptic and antifungal agent, a deoxygenating agent, an antioxidant, an antiseptic agent, a discoloration preventing agent, an antifoam agent, a flavor, and solvents other than alcohol and water.

It goes without saying that any configuration shown in an embodiment in the present description is merely one example, and various modifications are possible without departing from the scope of the present invention.

### EXAMPLE OF STICKING TO SKIN

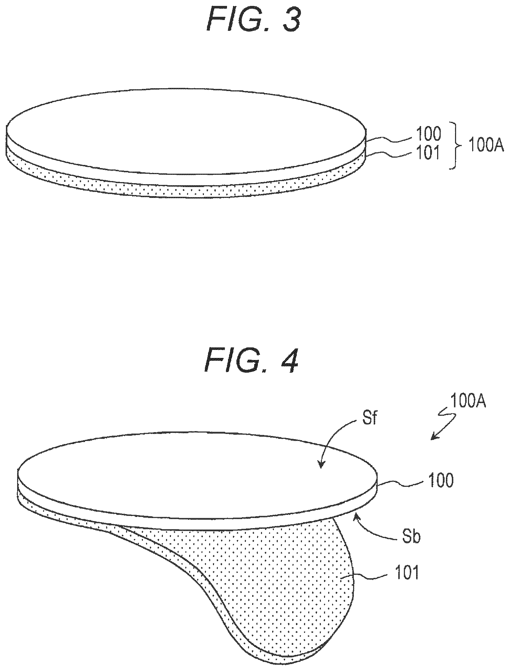

FIG. 3 and FIG. 4 show an example of sticking the cellulose membrane according to an embodiment of the present disclosure to skin. As shown in FIG. 4, cellulose membrane 100 according to an embodiment of the present disclosure can be provided in the form of a laminate having a cellulose membrane and a protective layer. Multilayer sheet 100A shown in FIG. 4 has cellulose membrane 100, and protective layer 101 disposed on one of principal faces of cellulose membrane 100. Cellulose membrane 100 is composed of regenerated cellulose having a weight average molecular weight of not less than 150,000. It goes without saying that FIG. 3 and FIG. 4 show multilayer sheet 100A merely schematically, and actual dimensions are not precisely reflected. For example, thicknesses of cellulose membrane 100 and protective layer 101 are exaggerated in FIG. 3 and FIG. 4. Also in other drawings of the present disclosure, the cellulose membrane or the like may be depicted in a dimension or a shape that is different from the actual dimension or shape for convenience of illustration.

In this example, cellulose membrane 100 has a generally circular shape. Cellulose membrane 100 shown in FIG. 4 can have a diameter of, for example, about 3 mm. Naturally, the shape of cellulose membrane 100 is not limited to the example shown in FIG. 3, but can be an ellipse, a polygon or indeterminate form. Cellulose membrane 100 and protective layer 101 may be different in size.

Reference is made to FIG. 4. Cellulose membrane 100 has principal faces Sf and Sb, and protective layer 101 is disposed on a side of principal face Sb herein. Protective layer 101 is a sheet or a nonwoven fabric of, for example, polyethylene, polypropylene, polyethylene terephthalate, nylon, acryl resin, polycarbonate, polyvinyl chloride, acrylonitrile-butadiene-styrene (ABS) resin, polyurethane, synthetic rubber, cellulose, Teflon (registered trademark), aramid, and polyimide, or sheet-like metal, glass, and the like. The whole or part of the surface of the sheet or the nonwoven fabric may be subjected to a chemical or physical surface treatment. In this example, protective layer 101 also has a circular shape likewise cellulose membrane 100. However, it is not necessary that cellulose membrane 100 and protective layer 101 are coincident in shape. For example, a plurality of cellulose membranes 100 can be disposed on single protective layer 101. Protective layer 101 in multilayer sheet 100A is not a support for keeping the shape of cellulose membrane 100.

As schematically shown in FIG. 4, protective layer 101 is formed so as to be peelable from principal face Sb of cellulose membrane 100. Cellulose membrane 100 has a tensile strength of, for example, not less than 23 MPa, and can keep the shape even in a state that protective layer 101 is peeled off.

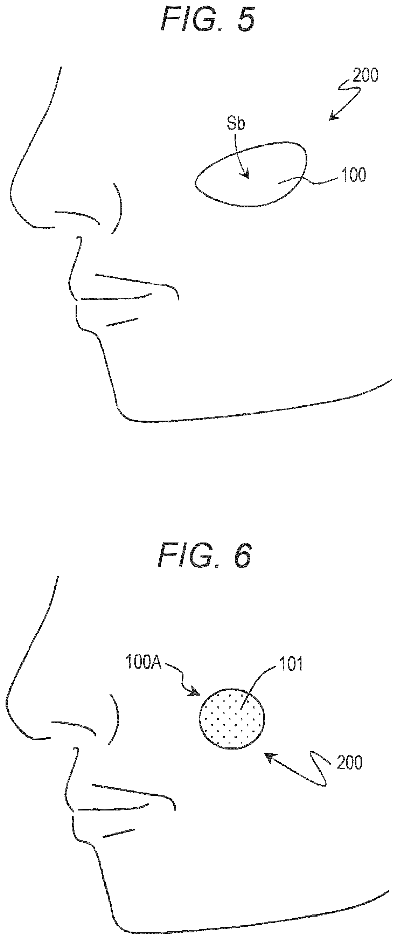

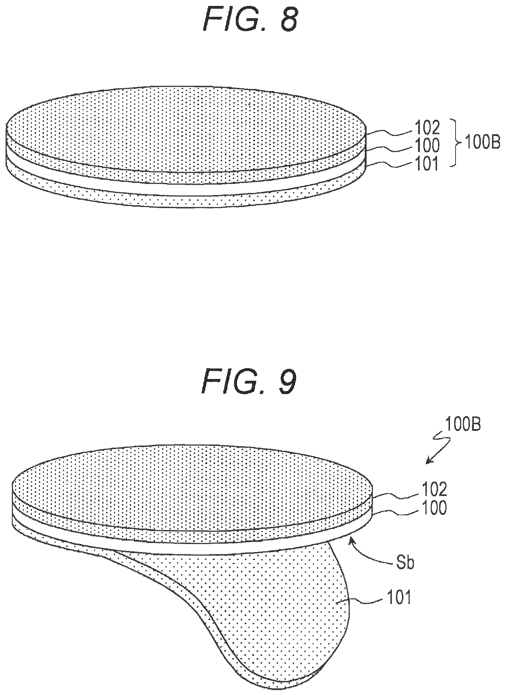

Next, in a condition that principal face Sf of cellulose membrane 100 is opposed to skin 200, multilayer sheet 100A is brought into contact with skin 200, and thus multilayer sheet 100A is stuck to skin 200 as shown in FIG. 6. Further, as shown in FIG. 8, protective layer 101 is peeled off from principal face Sb of cellulose membrane 100. By peeling off protective layer 101 from cellulose membrane 100, it is possible to leave cellulose membrane 100 on skin 200 (see FIG. 5).

Other protective layers may be provided on principal face Sf of cellulose membrane 100. FIG. 8 shows another example of the multilayer sheet. Multilayer sheet 100B shown in FIG. 8 has second protective layer 102 on a principal face on an opposite side of the principal face on which protective layer 101 is disposed, of principal faces of cellulose membrane 100. The material that forms protective layer 102 may be the same as or different from the material of protective layer 101. The size of protective layer 102 may be different from the size of cellulose membrane 100 or protective layer 101. Typically, also protective layer 102 can be peeled off from cellulose membrane 100 likewise protective layer 101. Existence of protective layer 102 further facilitates handling of cellulose membrane 100.

When such multilayer sheet 100B is used, first, protective layer 101 is peeled off from cellulose membrane 100 as shown in FIG. 9. Removal of protective layer 101 results in exposure of principal face Sb of cellulose membrane 100. Then, exposed principal face Sb is opposed to skin 200.

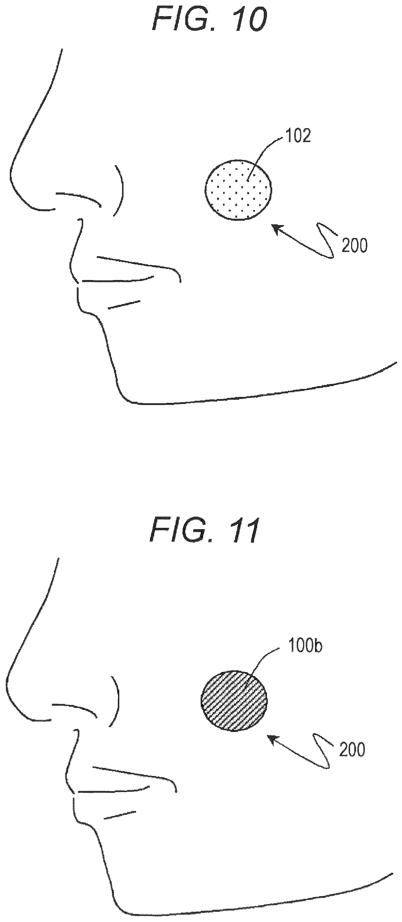

Next, as shown in FIG. 10, a laminate of cellulose membrane 100 and second protective layer 102 is stuck to skin 200. Then, protective layer 102 is peeled off from the other of principal faces (principal face opposite to principal face Sb) of cellulose membrane 100. By peeling off protective layer 102, it is possible to leave cellulose membrane 100 on skin 200.

The cellulose membrane of the present disclosure may be at least partly colored. FIG. 11 schematically shows a state that colored cellulose membrane 100b is stuck to skin 200. According to the aforementioned illustrative production method, a transparent cellulose membrane is typically obtained. By using cellulose membrane 100b that is colored with a skin-like color, it is possible to cover a blotch, a mole, a scar and the like on skin 200 with cellulose membrane 100b and to make them be inconspicuous. For example, cellulose membrane 100 stuck on a scar can function as a protective sheet that protects the skin from external stimulation. Cellulose membrane 100 may retain a component intended for medical care. Alternatively, the cellulose membrane can be usable as a decorating sheet such as a tattoo sticker when the cellulose membrane is provided with a pattern and a color by printing or the like.

### EXAMPLES

Hereinafter, the present invention will be described in more detail by way of examples; however, the present invention is not limited by the following examples.

First, Example 1 will be described.

### PREPARATION OF COSMETIC AGENT

Cosmetic agents were prepared by mixing materials in the mixing ratios shown in Table 1.

A cosmetic agent of Example 1 was prepared by adding 5% by weight of concentrated glycerin for cosmetics available from Kao Corporation and 5% by weight of Zemea Select propanediol available from Iwase Cosfa Co., Ltd. to 90% by weight of ultrapure water.

### PREPARATION OF CELLULOSE MEMBRANE

A cellulose membrane having each thickness was prepared in the following procedure. First, cellulose derived from bleached pulp made of wood and having a purity of not less than 95% was prepared.

By dissolving cellulose derived from bleached pulp in an ionic fluid, a cellulose solution was prepared. As the ionic fluid, 1-ethyl-3-methylimidazolium diethylphosphate (available from TOKYO CHEMICAL INDUSTRY CO., LTD.) was used. Next, a glass substrate with a flat surface and having a contact angle with water of 34° was prepared. The contact angle was determined according to the θ/2 method using an automated contact angle meter DM-501 manufactured by Kyowa Interface Science Co., LTD. Then, by applying the cellulose solution on the surface of the glass substrate by gap coating, a liquid membrane was formed on the glass substrate. At that time, by adjusting the size of the gap, the thickness of the cellulose membrane was adjusted.

After formation of the liquid membrane, the glass substrate and the liquid membrane were sufficiently left to stand in an environment of 25°C and 30 to 40%RH, to cause gelation of the liquid membrane, and thus a polymer gel sheet was obtained. Thereafter, the polymer gel sheet was washed with water to remove the ionic fluid from the polymer gel sheet. (At this time, by immersing the glass substrate and the polymer gel sheet in ultrapure water, and replacing the ultrapure water a plurality of times, water washing of the polymer gel sheet was carried out.)

The polymer gel sheet after washing was held with tweezers and removed from the glass substrate in the ultrapure water, and placed on a nonwoven fabric. The polymer gel sheet on the nonwoven fabric was removed from the ultrapure water, and the polymer gel sheet was dried by heating at 70°C. By peeling off the dried polymer gel sheet from the nonwoven fabric, a cellulose membrane was obtained. The cellulose membrane had a shape of approximately 5 cm × 5 cm, and transparent appearance.

Thickness d of the cellulose membrane placed on the glass substrate was measured with a stylus profiling system DEKTAK (registered trademark) manufactured by Bruker Nano Incorporated, and the obtained cellulose membrane had a thickness of about 890 nm.

A weight average molecular weight of the obtained cellulose membrane measured by Gel Permeation Chromatography (GPC)-Multi Angle Light Scattering (MALS) method was about 224,000.

For the measurement, a liquid delivery unit LC-20AD manufactured by Shimadzu Corporation was used, and as detectors, a differential refractometer Optilab rEX and a multi-angle light scattering detector DAWN HELEOS manufactured by Wyatt Technology Corporation were used. As a column, TSKgel α-M manufactured by TOSOH CORPORATION was used, and as a solvent, dimethyl acetamide to which lithium chloride was added in 0.1 M was used. The measurement was conducted at a column temperature of 23°C, and a flow rate of 0.8 mL/min.

A water vapour transmission rate of the cellulose membrane measured by the method according to JIS K 7129-C was 3.8×10⁴g/m²·24h.

### EVALUATION OF STICKING OF CELLULOSE MEMBRANE BY COSMETIC AGENT

Regarding the skin sticking performance of the cellulose membrane by the prepared cosmetic agent, general evaluation was made based on the results of evaluation of appearance when the cellulose membrane was stuck to skin, adhesiveness during continuous attachment, and evaluation of the adhesion against frictional stimulation. Evaluation was made for three female subjects aged 30s and 40s, and an average was calculated.

### EVALUATION OF APPEARANCE WHEN CELLULOSE MEMBRANE WAS STUCK TO SKIN

In the following manner, appearance when the cellulose membrane was stuck to skin was evaluated. First, 10 µl of the cosmetic agent was applied on skin of the inner side of an upper arm and extended into a circle measuring 1.5 cm in diameter, and on the cosmetic agent, a cellulose membrane of 1 cm² square was stuck. The appearance of the sheet on the skin directly after sticking was scored according to the following criteria. The appearance was evaluated in a room with a general fluorescent lamp.

Score 4: The sheet is invisible or slightly visible depending on the angle of the light in a place where the light of the fluorescent lamp directly comes.

Score 3: The sheet is visible from any angle in a place where the light of the fluorescent lamp directly comes, but there are no color change, and wrinkle and bracing in the sticking part.

Score 2: The sheet is visible from any angle in a place where the light of the fluorescent lamp does not directly come, but there are no color change, and wrinkle and bracing in the sticking part.

Score 1: The sheet is visible in a place where the light of the fluorescent lamp does not directly come, and there are color change, and wrinkle and bracing in the sticking part.

### EVALUATION OF CONTINUOUS ADHESION

In the following manner, appearance when the cellulose membrane was stuck to skin was evaluated. First, 10 µl of the cosmetic agent was applied on skin of the inner side of an upper arm and extended into a circle measuring 1.5 cm in diameter, and on the cosmetic agent, a cellulose membrane of 1 cm² square was stuck. After a lapse of 3 hours in that state, occurrence of peeling of the sample from the skin was scored by a visual inspection according to the following criteria.

Score 4: No peeling observed.

Score 3: Slight peeling observed in an end of the sample.

Score 2: Peeling of not more than 30% of the area of the sample observed, but the sample existing on the skin

Score 1: Peeling of not less than 30% of the area of the sample observed, or the sample fell off the skin

### EVALUATION OF ADHESION AGAINST FRICTIONAL STIMULATION

In the following manner, appearance when the cellulose membrane was stuck to skin was evaluated. First, 10 µl of the cosmetic agent was applied on skin of the inner side of an upper arm and extended into a circle measuring 1.5 cm in diameter, and on the cosmetic agent, a cellulose membrane of 1 cm² square was stuck. After a lapse of not less than 30 minutes in that state, the sample was rubbed with a swab, and occurrence of peeling was scored according to the following criteria.

Score 4: No peeling occurred after rubbing of 11 times or more.

Score 3: No peeling occurred at the point of rubbing of 10 times.

Score 2: Part of the sample peeled off at the point of rubbing of 10 times, but the sample still existing on the skin.

Score 1: Part of the sample peeled off at the point of rubbing of two or three times, or the sample fell off the skin.

### GENERAL EVALUATION

General judgement was made when the cellulose membrane was stuck to skin according to the following criteria.
⊙: 4 points or more in every item
○: 3 points or more in every item
Δ: 2 points or more in every item
×: 1 point or less in any one of items

Next, Examples 2 to 19 will be described.

Cosmetic agents were prepared in the same manner as in Example 1 according to the mixing ratios shown in Table 1 to Table 4. Propylene glycol for cosmetics available from ADEKA CORPORATION was used as propylene glycol, DPG-DF available from ADEKA CORPORATION was used as dipropylene glycol, 1,3-butylene glycol available from Iwase Cosfa Co., Ltd., dehydrated ethanol available from Taiyo Pharmaceutical Co., Ltd was used as ethanol, Hyaluronsan HA-LQ available from Kewpie Corporation was used as sodium hyaluronate, and NOMUCOAT Z available from Nisshin Oillio Group, Ltd. was used as xanthan gum.

Regarding the cellulose membrane, the same cellulose membrane as used in Example 1 was used in Examples 2 to 16. In Examples 17 and 18, a cellulose membrane as prepared in the above method except for the size of the gap in forming a liquid membrane on the glass substrate was used. The thickness was 295 nm in Example 17, the thickness was 560 nm in Example 18, and the thickness was 1319 nm in Example 19.

Evaluation of sticking of the cellulose membrane by the cosmetic agent was conducted in the same manner as in Example 1.

The results of attempts for preparing a self-supporting cellulose membrane in various conditions are shown.

A cellulose membrane having a thickness of 17 nm was prepared in the same manner as described above by adjusting the concentration of the cellulose solution and the size of the gap. The cellulose membrane was difficult to be picked up with tweezers without deformation of the membrane, and was proved to be a membrane not capable of self-supporting.

A cellulose membrane having a thickness of 93 nm was prepared in the same manner as described above by adjusting the concentration of the cellulose solution and the size of the gap. The membrane was a self-supporting membrane.

First, as cellulose to be solved in an ionic fluid, filter paper made of wood and having a purity of cellulose of not less than 99% was prepared. A membrane having a thickness of 190 nm was prepared in the same manner as in the case where the cellulose was cellulose derived from breached pulp except that 1-ethyl-3-methylimidazolium diethylphosphate (available from TOKYO CHEMICAL INDUSTRY CO., LTD.) was used as the ionic fluid for dissolving cellulose. The membrane was a self-supporting membrane. The obtained membrane had a weight average molecular weight Mw of about 152,000.

As the cellulose to be dissolved in an ionic fluid, microcrystalline cellulose (product name: Avicel (registered trademark)) was prepared. Preparation of a cellulose membrane was attempted in the same manner as in the case where the cellulose was derived from breached pulp. However, the polymer gel sheet was finely crushed during the course of peeling off the polymer gel sheet from the glass substrate and removing the polymer gel sheet from the ultrapure water, and the membrane was proved to be a membrane not capable of self-supporting. A regenerated cellulose piece left with a certain degree of size had a weight average molecular weight Mw of about 30,800.

This result reveals that a self-supporting cellulose membrane has a weight average molecular weight of about not less than 150,000.

### COMPARATIVE EXAMPLES

Next, Comparative Examples 1 to 5 will be described.

Cosmetic agents were prepared by mixing materials according to the mixing ratios shown in Table 3 to Table 4. In Comparative Example 1, a sample containing only 100% by weight of ultrapure water was prepared. A cosmetic agent of Comparative Example 2 was prepared by adding and mixing 15% by weight of ethanol to 85% by weight of ultrapure water. Also cosmetic agents of Comparative examples 3, 4 were prepared in the same manner as in Comparative Example 2 according to the mixing ratios of Table 3. For Comparative Examples 1 to 4, preparation and evaluation of the cellulose membrane were conducted in the same procedure as in Example 1. For Comparative Example 5, the cellulose membrane was prepared in the same manner as in Example 1 except for the size of the gap in forming a liquid membrane on the glass substrate, and the cellulose membrane had a thickness of 2100 nm.

Evaluation of sticking of the cellulose membrane by the cosmetic agent was conducted in the same manner as in Example 1.

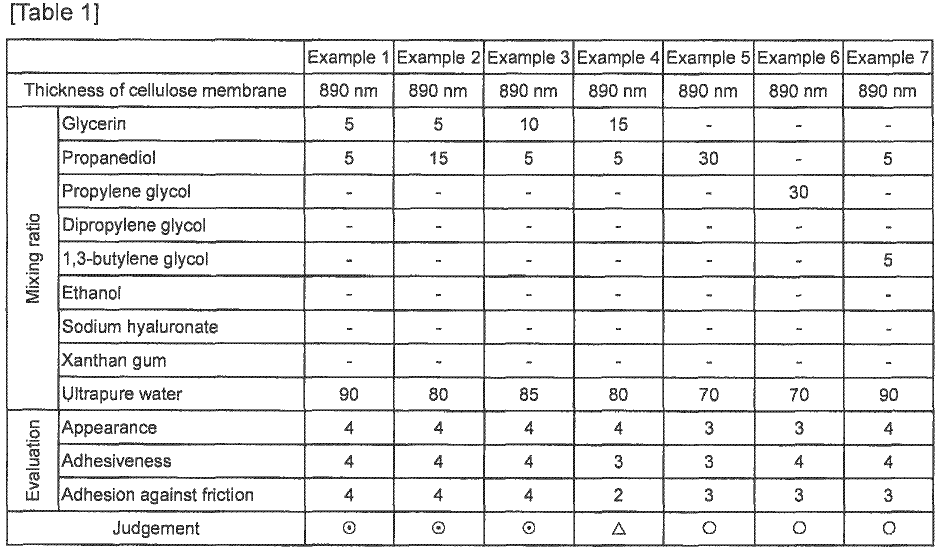

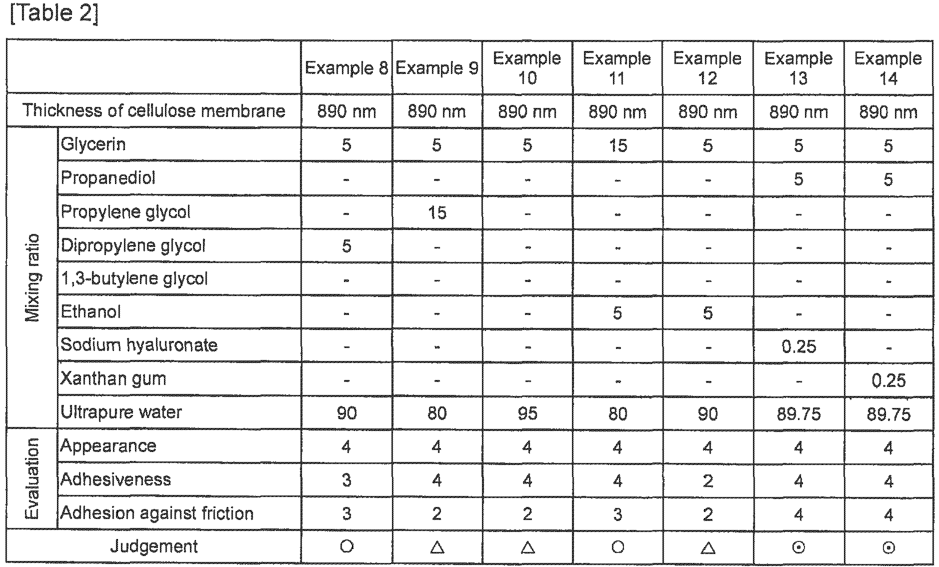

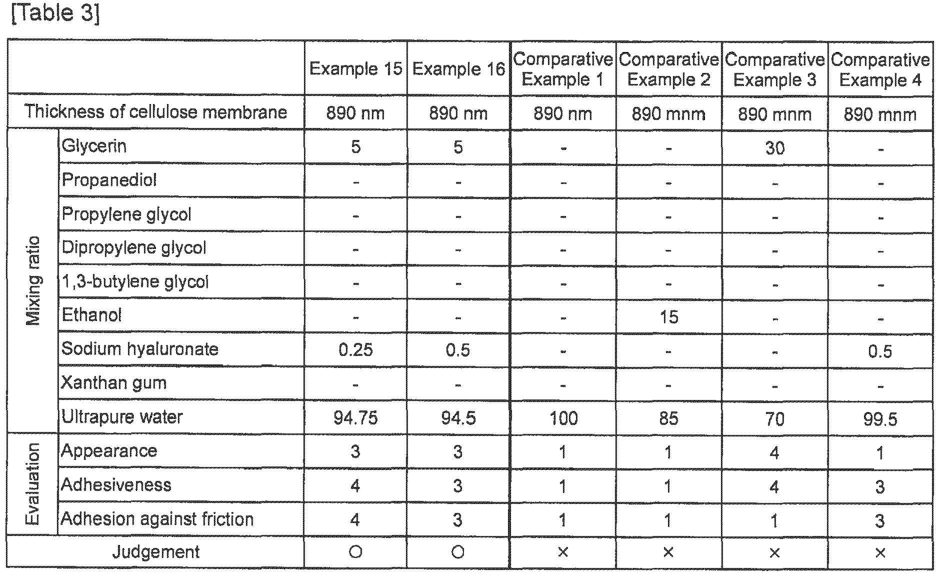

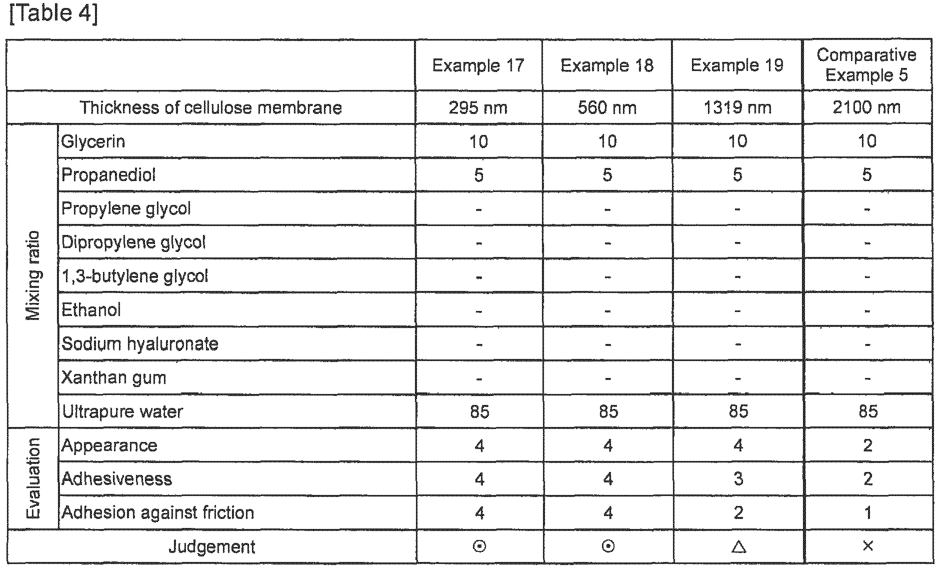

Table 1 reveals that the appearance when the cellulose membrane was stuck to skin, the long-term adhesiveness to skin, and the adhesion against frictional stimulation vary by the present invention.

When only water was used as in Comparative Example 1, the score was 1 point or less in any items. In contrast, when a cosmetic agent containing water and 15% by weight or less of glycerin was used as in Examples 10, 11, and 12, the appearance when the cellulose membrane was stuck to skin, the long-term adhesiveness to skin, and the adhesion against frictional stimulation were improved, and the score was 2 points or more in every item.

In the case of a cosmetic agent containing water and propanediol or propylene glycol which is a diol alcohol as shown in Examples 5 and 6, the score was 3 points or more in every item.

When a cosmetic agent containing water, glycerin, and propanediol or propylene glycol or dipropylene glycol which is a diol alcohol was used as shown in Examples 1, 2, 3, 4, 8, 9, 13, and 14, the score was 2 points or more in every item.

In the case of containing water and propanediol and 1,3-butylene glycol which are two kinds of diol alcohols as shown in Example 7, the score was 3 or more in every item.

The results of Examples 1, 2, 3, 13, and 14 revealed that in the case of using a combination of 5 to 10% by weight of glycerin and 5 to 15% by weight of propanediol, the score was 4 points or more in every item.

In the case of containing 30% by weight of glycerin as shown in Comparative Example 3, the items of the appearance and the long-term adhesiveness were improved, but the result in the item of the adhesion against frictional stimulation was low. The reason for this is as follows: glycerin is difficult to be dry because glycerin is a trihydric alcohol having many hydroxyl groups and has high boiling point and hygroscopicity, and when the mixing rate of glycerin in the cosmetic agent is high, a large amount of glycerin remains for a long time between the cellulose membrane and the skin, so that the followability of the cellulose membrane to the skin is impaired, the adhesion deteriorates, and the cellulose membrane becomes easy to be peeled off by frictional stimulation.

Also, as shown in Comparative Examples 2 and 4, in the case of containing only ethanol as a monohydric alcohol which is a component other than a polyhydric alcohol, or sodium hyaluronate which is a water-soluble polymer and a moisturizer, improvement was not observed in any of items.

These results reveal that a cosmetic agent containing one or more kinds of polyhydric alcohols and having a content of glycerin of not more than 15% by weight is useful for sticking a cellulose membrane to skin for a long time without giving any strange feeling of appearance. In particular, when a combination of 5% by weight to 10% by weight of glycerin and 5% by weight to 15% by weight of propanediol is used, the effect is higher as compared with cosmetic agents of other mixing ratios.

When the same cosmetic agent was used as shown in Table 2, the score was 4 points or more in every item for a thickness of not more than 890 nm, but the score was 2 points or more for a thickness of 1,319 nm, which means characteristic deterioration, and the characteristic was 1 point or less for a thickness of 2,100 nm in Comparative Example 5. This leads to the conclusion that the thickness of the cellulose membrane for stably sticking to skin for a long time is not more than 1,300 nm.

### INDUSTRIAL APPLICABILITY

The method for sticking a sheet to skin according to an embodiment of the present disclosure is a method capable of sticking a sheet to skin without using an adhesive, and enables stable sticking a sheet to skin for a long time without giving a skin stress and strange feeling of appearance. For example, the method can be used for sticking of a skin protective film or a skin care film intended for cosmetics or medical care. Also it is possible to make the cellulose membrane retain a component that acts on living organism or protects living organism such as a cosmetic component, or the cellulose membrane may be provided with color or a pattern, and the method for sticking a sheet to skin according to the present disclosure can also be used for sticking a functional sheet for protection or decoration as well as for cosmetics or medical cares, for example.

## Claims

1. A method for sticking a sheet, the method comprising:
applying a cosmetic agent containing water and at least one kind of polyhydric alcohol to skin; and
placing a self-supporting cellulose membrane having a thickness of not less than 20 nm and not more than 1,300 nm and containing regenerated cellulose having a weight average molecular weight of not less than 150,000 on the skin,
wherein
an amount of glycerin of the polyhydric alcohol contained in the cosmetic agent is not less than 0% by weight and not more than 15% by weight.

2. The method for sticking a sheet to skin according to claim 1, wherein the self-supporting cellulose membrane has a bulk density of not less than 0.3 g/cm³ and not more than 1.5g/cm³.

3. The method for sticking a sheet to skin according to claim 1 or 2, wherein the polyhydric alcohol is a diol alcohol.

4. The method for sticking a sheet to skin according to claim 3, wherein the diol alcohol is propanediol.

5. The method for sticking a sheet to skin according to any one of claims 1 to 4, wherein the cosmetic agent contains, as the polyhydric alcohol, 5% by weight to 15% by weight of propanediol and 5% by weight to 10% by weight of glycerin.

6. The method for sticking a sheet to skin according to claim 3, wherein the diol alcohol is propylene glycol.

7. The method for sticking a sheet to skin according to claim 3, wherein the diol alcohol is dipropylene glycol.

8. The method for sticking a sheet to skin according to claim 3, wherein the diol alcohol includes two kinds of propanediol and 1,3-butylene glycol.

9. The method for sticking a sheet to skin according to any one of claims 1 to 8, wherein the cosmetic agent further contains ethanol.

10. The method for sticking a sheet to skin according to any one of claims 1 to 9, wherein the cosmetic agent further contains a water-soluble polymer.

11. A cosmetic agent for sticking a cellulose membrane to skin, the cosmetic agent containing:
water;
5% by weight to 15% by weight of propanediol; and
5% by weight to 10% by weight of glycerin.
